# EUROPEAN PATENT SPECIFICATION

(11) **EP 1 753 505 B1**
(45) Date of publication and mention of the grant of the patent: **07.10.2009**
(21) Application number: 04735435.2
(22) Date of filing: 28.05.2004
(51) Int. Cl.: A61N 1/372, A61N 1/375, H01Q 9/43, H01Q 1/22

(54) **IMPLANTABLE MEDICAL DEVICE**
IMPLANTIERBARE MEDIZINISCHE VORRICHTUNG
DISPOSITIF MEDICAL IMPLANTABLE

(43) Date of publication of application: 21.02.2007
(73) Proprietor: St. Jude Medical AB, 175 84 Järfälla (SE)
(72) Inventor: LIM, Wisit, Palmdale, California 93551 (US); SAHABI, Kavous, Winnetka, California 91306 (US); SKÖLDENGEN, Niklas, S-SE-187 50 Täby (SE); SNITTING, Tomas, SE-113 38 Stockholm (SE); HJELM, Stefan, S-SE-746 91 Bålsta (SE)
(86) International application number: PCT/SE2004/000831
(87) International publication number: WO 2005/115540

(56) References cited:
- EP-A1- 1 582 235
- US-A1- 2004 027 306
- US-B1- 6 240 317
- US-B2- 6 675 045

## Description

### FIELD OF INVENTION

The present invention relates to an implantable medical device.

### DESCRIPTION OF RELATED ART AND BACKGROUND OF THE INVENTION

In an implantable medical device, such as a cardiac pacemaker, an implantable cardioverter/defibrillator, or an insulin dispenser, telemetry is used to e.g. change or modify operation characteristics of the implantable device or to readout data from the implantable medical device to monitor its function or to achieve information of the patient having the device implanted. Telemetry systems for implantable medical devices are used for communication between the implantable device and an external programmer device.

Earlier telemetry systems used a carrier having a rather low frequency for communication between an antenna of the implantable device and an antenna of the external programmer device, which were inductively coupled to each other. Due to the limited operating distance of this technique, the exterior antenna had to be located in close proximity to the implantable device, typically within a few inches. Further, the communication suffered from low transmission data rate.

Recently, telemetry systems using a radio frequency data link operating at a much higher frequency, around 400 MHz, have been proposed, which enable two improvements to be made. Firstly, the antenna efficiency can be improved allowing extended range between the pacemaker and the external antenna. Secondly, the transmission data rate can be improved. Even higher frequencies can be used such as those within the ISM-band at 2400-2485.5 MHz.

Pacemakers including antenna arrangements for telemetry are disclosed in US 6,169,925 B1, US 5,342,408, US 5,313,953, and FR 2 559 671.

US 6,169,925 B1 to Villaseca et al. discloses an implantable medical device utilizing such electromagnetic radiation to allow communication over a large distance. The device has in one embodiment thereof an external antenna protruding from a dielectric header or connector block of the device. The antenna is provided in the form of a wire surrounded by an insulative coating, and in the context of a device operating in the vicinity of 400 MHz, for example, the length of the wire may be approximately 8 cm.

In another embodiment of said patent the antenna takes the form of a length of coaxial cable having its center conductor coupled to a feed-through which is in turn coupled to a transceiver within the device and having a braided shield coupled to the device enclosure, which is typically fabricated of a conductive material such as stainless steel or titanium. The braided shield extends over only a portion of the length of the antenna. This antenna, which may be approximately 12 cm in length, is provided within a dielectric header of the device.

Electrical therapy leads are arrangable in close vicinity to the antennas.

US-A-2004 0 027 306 discloses an implantable cardiac rhythm management device with a telemetry antenna in an extended header.

EP-A-1 582 235, that was published after the filing date of the present application, discloses an implantable medical device with a conductive housing and a conductive portion on the header, wherein the housing and the conductive portion are electrically connected to each other and jointly act as an antenna.

### SUMMARY OF THE INVENTION

The above-described antennas are space consuming and do not use the available space optimally. The first above-described embodiment of US 6,169,925 B1 discloses an antenna that protrudes from the housing a rather long distance. The second embodiment discloses an antenna that is located within a dielectric header, which thus has to be of considerable size.

Further, the first above-described embodiment of US 6,169,925 B1 seems not to have an optimum mechanical structure, and both the embodiments seem to be complex and costly to produce.

The relatively strong electromagnetic coupling between the antenna and the electrical therapy lead due to the antenna having an open end close to the therapy lead could be a problem since it may i.a. lead to impedance mismatch of the antenna.

It is thus an object of the present invention to provide an implantable medical device that overcomes the above-mentioned problems.

In this respect there is a particular object of the invention to provide such an implantable medical device, which exhibits an overall improved antenna performance in comparison with implantable medical devices of the prior art. Higher antenna performance implies increased usable range for an exterior antenna.

A further object of the invention is to provide such an implantable medical device, wherein the antenna operation does not essentially influence the operation of other electrical equipment in its vicinity, such as e.g. therapy or sensing leads, and wherein the antenna operation is not essentially influenced by the operation of other electrical equipment.

A still further object of the invention is to provide such an implantable medical device, which has a minimum number of feed-throughs in the housing thereof.

A yet further object of the invention is to provide such an implantable medical device, in which the antenna is easy to manufacture to a low cost, easy to tune, and which antenna enables an efficient use of available space.

A still further object of the invention is to provide such an implantable medical device, which is reliable, and particularly mechanically durable.

Implantable medical devices as claimed in the appended patent claims attain these objects, among others.

Preferably, the implantable medical device comprises at least one opening for receiving and supporting at least one lead, such as a therapy or sensing lead, which is connectable to the electric circuitry of the implantable medical device.

A major portion of the conductive structure or the entire conductive structure is preferably provided on a surface of the dielectric header, which may advantageously be an outer surface of the implantable medical device, i.e. a surface to be in tissue contact after implantation.

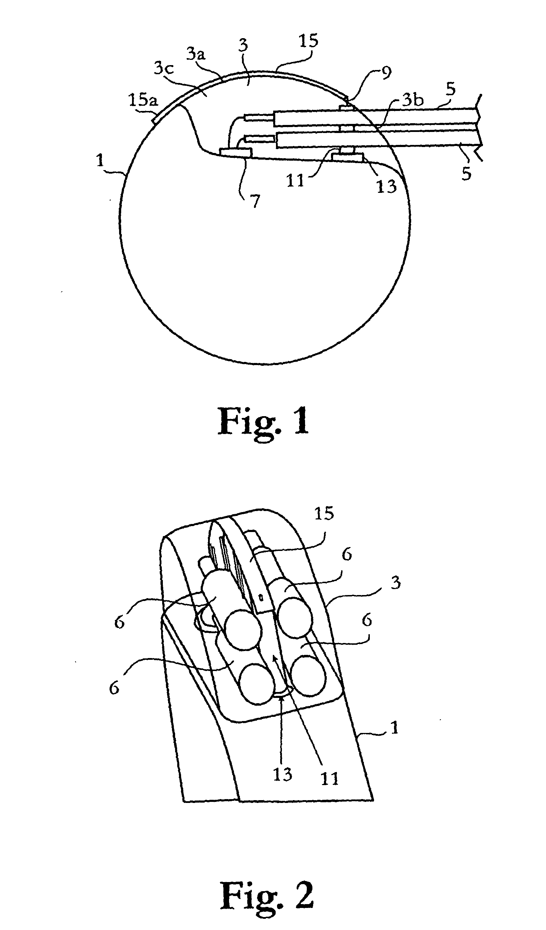

Further characteristics of the invention and advantages thereof, will be evident from the following detailed description of preferred embodiments of the present invention given hereinafter and from the accompanying Figs. 1-6, which are given by way of illustration only, and shall thus not limit the scope of the present invention.

In the description it is to be understood that the antenna of the present invention is operable to transmit or receive radio frequency signals. Even if a term is used herein that suggests one specific signal direction it is to be appreciated that such a situation can cover that signal direction and/or its reverse.

### BRIEF DESCRIPTION OF THE DRAWINGS

Fig. 1 illustrates schematically, in a top view, an implantable medical device according to a preferred embodiment of the present invention.
Figs. 2-3 illustrate, in enlarged perspective views, portions of the implantable medical device of Fig. 1.
Figs. 4-5 illustrate schematically, in top views, implantable medical devices according to further preferred embodiments of the invention.
Fig. 6 illustrates schematically, in side view, an implantable medical device according to still a further preferred embodiment of the invention.

Throughout the Figures similar parts and components, and portions thereof, are denoted by identical reference numerals.

### DETAILED DESCRIPTION OF PREFERRED EMBODIMENTS

A first preferred embodiment of an implantable medical device of the present invention will be described with reference to Figs. 1-3. The implantable medical device may e.g. be a pacemaker, a defibrillator, or other medical equipment including a telemetry link, preferably a high or radio frequency telemetry link.

The implantable medical device comprises an electrically conductive, hollow and hermetically sealed housing 1, electric circuitry for the operation of the implantable medical device and radio frequency circuitry for transmitting and/or receiving radio frequency telemetry signals. The electric circuitry and the radio frequency circuitry are interconnected and are arranged in the electrically conductive housing in a common space or in separate compartments, which may be shielded from each other.

A transparent electrically insulating or dielectric header portion 3 is mounted to the housing 1, wherein the header portion 3 advantageously is a molded component, e.g. an injection molded part. The header portion 3 may support four therapy or sensing leads 5, which are connected to the electric circuitry in the housing 1 via at least one electrically insulating and hermetic feed-through 7 in the wall of the housing 1. In order to support the leads 5 and provide space for electrical connections, the header portion 3 is provided with openings 6 and channels.

Note that only two of the leads 5 are visible in Fig. 1, and only the four openings for the leads are visible in Figs. 2 - 3.

Furthermore, the implantable medical device comprises an antenna device for transmitting and/or receiving the radio frequency telemetry signals, which includes an electrically conductive structure 15 provided on a surface of the electrically insulating header portion 3, preferably a surface 3a that constitutes an outer surface of the implantable medical device, and an antenna feeding 9, 11 operatively interconnected between the radio frequency circuitry of the implantable medical device and the electrically conductive structure 15.

The electrically conductive structure 15 is galvanically connected to the electrically conductive housing 1, and the electrically conductive structure 15 and the electrically conductive housing 1 are jointly adapted to operate as a transmitting and/or receiving antenna for the radio frequency telemetry signals. It shall be noted that the electrically conductive structure 15 typically is in physical contact with the electrically conductive housing 1. However, the electrically conductive structure 15 may be provided with discrete electrical components such as e.g. capacitors, inductors, diodes, and the like.

The antenna feeding 9, 11 is provided with an electrically insulating and hermetic feed-through 13 in the wall of the housing 1 of the implantable medical device. In the illustrated embodiment the antenna feeding 9, 11 comprises center 9 and shield 11 conductors of a coaxial cable, which both are fed through the wall of the housing 1 and guided by a through hole in the electrically insulating header portion 3 to an outer surface of the implantable medical device. The center conductor 9 is at this position electrically connected to the electrically conductive structure 15, whereas the shield conductor 11 is not connected thereto.

In an alternative non-illustrated variant of the embodiment the shield conductor 11 is not fed through the wall of the housing 1, but the feed-through 13 is provided only to feed the center conductor 9 through the wall, which is then guided by the through hole to connect with the electrically conductive structure 15. The conductor may be thicker than an ordinary center conductor of a coaxial cable in this variant. The portion of the center conductor 9, which is located outside the housing 1, will constitute an integral part of the radiating antenna of the implantable medical device.

Alternatively, the conductive structure 15 is guided by the through hole to connect with the center conductor 9 adjacent the feed-through 13.

The shield conductor 11 is advantageously connected to the housing 1 in the vicinity of the feed-through 13.

It shall be appreciated that the feeding of the conductive structure 15 may be implemented in many other manners known in the art, e.g. by using a microstrip transmission line or by using a capacitively connected feeding.

Further, an impedance matching network or antenna tuning circuit may be provided for loading the antenna with a variable amount of inductance or capacitance to thereby adjust the effective electrical length of the antenna and match the antenna impedance to the impedance of the transmitter/receiver. In this manner, the reactance of the antenna may be tuned out so that the antenna forms a structure at the specified carrier frequency to efficiently transmit/receive radio frequency radiation.

The electrically conductive structure 15 is preferably a strip or a wire of a metallic material. It may be fabricated by means of printing a conductive pattern on a surface of the electrically insulating header portion 3. The strip or wire may have a length of at least 0.5 cm, preferably at least 1 cm, and most preferably at least 2 cm.

Preferably, the electrically conductive structure 15 is provided on a surface of the electrically insulating header portion 3, which constitutes an outer surface of the implantable medical device. In such a manner the electrically conductive structure 15 will be in physical contact with blood and tissue of the patient when the implantable medical device is implanted therein.

Yet, in order to use the available length of the electrically insulating header portion efficiently the through hole, wherein the antenna feeding 9, 11 is located, is provided in one end portion 3b of the electrically insulating header portion 3, whereas the electrically conductive structure 15 is galvanically connected to the electrically conductive housing 1 adjacent a second end portion 3c of the electrically insulating header portion 3, wherein the first and second end portions are located oppositely to each other. Hereby, the electrically conductive structure 15 can be provided along a major length of the insulating header portion 3.

The dielectric constant of matter such as blood and tissue may vary, which naturally affect the resonance frequency or wavelength of the antenna. The dielectric material is not important for the radiation performance as such, but tunes the resonance to a lower frequency than the one indicated by the physical length. At 400 MHz and a dielectric constant of 1, an effective wavelength to obtain resonance measures 72 cm, whereas at a dielectric constant of 65 the effective wavelength is 9.2 cm. The use of an electrically conductive radiating structure at the outer surface of the implantable medical device as being illustrated in Figs. 1-3 is obviously preferred to keep the dimensions small, especially when a frequency around 400 MHz is used.

Further, since the moisture content of the header portion 3 may vary with long term use, an antenna located within the header portion 3 would experience an impedance mismatch with time. This mismatch can be severe since a typical dielectric has a dielectric constant of about 2-4, whereas water has a dielectric constant of about 80. The provision of the electrically conductive structure 15 on the outer surface of the header portion 3 remedies this problem.

The implantable medical device of Figs. 1-3 is less complex and difficult to produce as compared to the prior art devices. Few feed-throughs are needed, the electrically conductive structure 15 is provided on the surface of the header portion 3, and the antenna is connected at the surface of the header portion 3. This enables the use of several different production methods.

The leads should preferably be located where they have as small influence on the antenna function as possible The inventors of the present invention has found that when the electrically conductive structure 15 is shorted to the housing 1 the antenna operation is less affected by the operation of the leads in close proximity of the antenna since the electromagnetic coupling between the antenna and the leads is weaker. This will lead to a more stable performance regardless of the number of leads that are used.

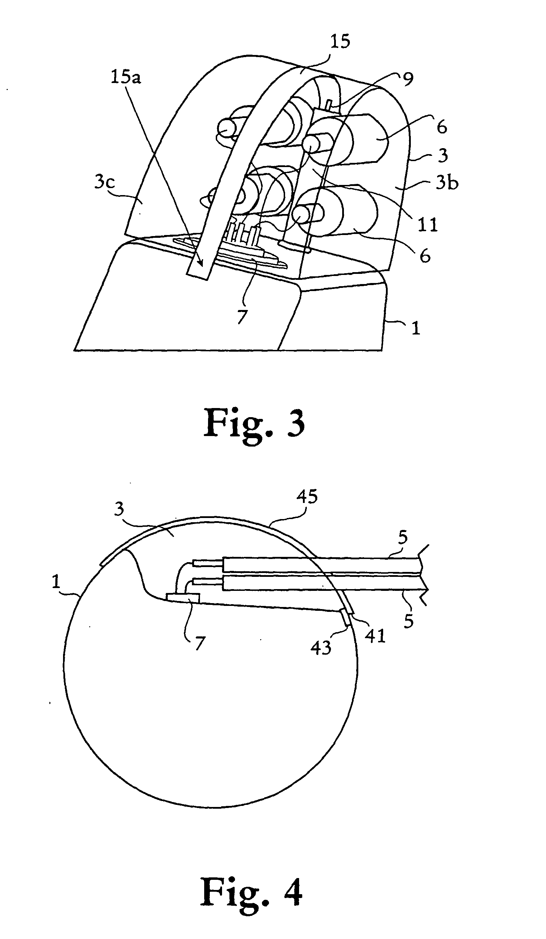

With reference next to Figs. 4-6 three alternative preferred embodiments of the implantable medical device of the present invention will be depicted.

The embodiment of Fig. 4 differs from the above described embodiment in that the electrically insulating and hermetic feed-through for the antenna feeding, here denoted by reference numeral 43, is provided in an interface between the housing 1 and the header portion 3. The antenna feeding comprises as above center 9 and shield 11 conductors of a coaxial cable, but only the center conductor, here denoted by 41, is fed through the wall of the housing 1 and connected to an electrically conductive structure 45 of the above described kind. In this embodiment no through hole for the antenna device in the electrically insulating header portion 3 is needed.

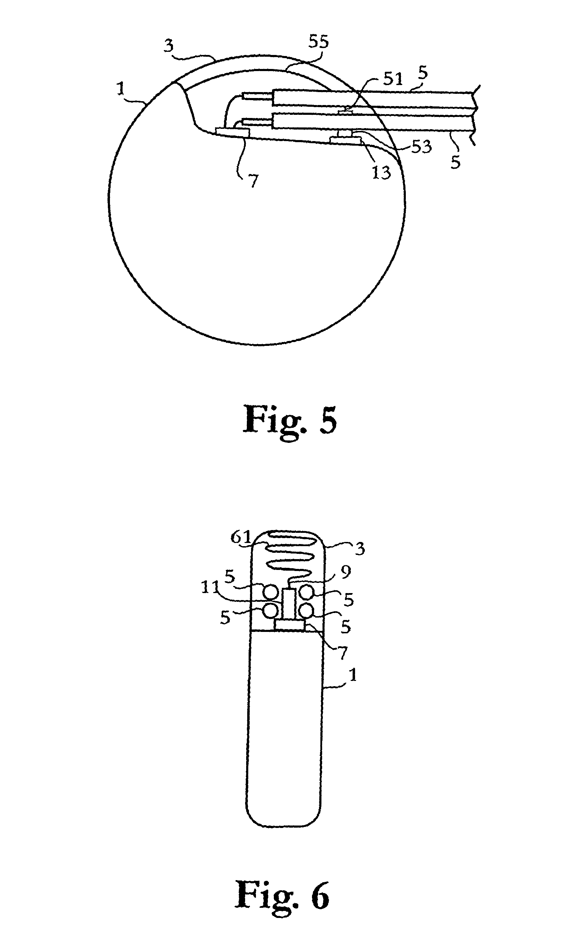

The embodiment of Fig. 5 differs from the embodiment of Figs. 1-3 in that the center and shield conductors of the antenna feeding, here denoted by 51 and 53, end within the header portion 3. The electrically conductive structure, preferably a wire and here denoted by 55, is connected to the end of the center conductor 51 *within* the header portion 3 and runs within the header portion 3 to an end thereof, at which position it is connected to the housing 1. To this end the header portion 3 is provided with a hole for the antenna feeding 51, 53, and a channel for the electrically conductive structure 55.

The embodiment of Fig. 6 differs from the embodiment of Figs. 1-3 only in that the electrically conductive structure, here denoted by 61, is meander shaped.

It shall be appreciated that the preferred embodiments described above are merely chosen to exemplify the present invention. Particularly, the electrically conductive structure of the present invention may taker other forms than those described above.

It shall still further be appreciated that the implantable medical device may be provided with two or more electrically conductive structure with separate feedings. Thus, such an implantable medical device may be adapted for transmitting and/or receiving radio frequency waves in at least two different frequency bands, e.g. both around 400 MHz and around 2.4 GHz.

## Claims

1. An implantable medical device comprising:
- an electrically conductive housing (1);
- electric circuitry for the operation of said implantable medical device and radio frequency circuitry for transmitting and/or receiving radio frequency signals, said electric circuitry and said radio frequency circuitry being interconnected and arranged within said electrically conductive housing (1);
- an electrically insulating header portion (3) arranged adjacent said electrically conductive housing;
- an electrically conductive structure (15, 45, 55, 61) is provided on a surface (3a) of, and/or within, said electrically insulating header portion (3);
- an antenna feeding (9, 11) is operatively interconnected between said radio frequency circuitry and said electrically conductive structure; and
- a through hole is provided in a first end portion (3b) of said electrically insulating header portion (3) and through which said antenna feeding (9, 11) and/or a minor portion of said electrically conductive structure (45) are/is guided, wherein
- said electrically conductive structure is galvanically connected to said electrically conductive housing adjacent a second end portion (3c) of said electrically insulating header portion, said first and second end portions being located oppositely to each other; and
- said electrically conductive structure and said electrically conductive housing are jointly adapted to operate as a transmitting and/or receiving antenna for said radio frequency signals.

2. The implantable medical device of claim 1 wherein said electrically insulating header portion (3) comprises at least one opening (6) for receiving at least one lead (5), and said electric circuitry is connectable to said at least one lead.

3. The implantable medical device of claim 2 wherein said housing is hermetically sealed, and a hermetic feed-through (7) is provided in a wall of said electrically conductive housing for guiding connections to said at least one lead (5) through said wall.

4. The implantable medical device of any of claims 1-3
wherein at least a major portion of said electrically conductive structure (15, 45, 61) is provided on the surface of said electrically insulating header portion.

5. The implantable medical device of claim 4 wherein the surface of said electrically insulating header portion, on which said at least major portion of said electrically conductive structure is provided, is an outer surface of said implantable medical device.

6. The implantable medical device of any of claims 1-5
wherein said electrically conductive structure (45) is provided on the surface of said electrically insulating header portion essentially in its entirety.

7. The implantable medical device of any of claims 1-6
wherein said electrically conductive structure is galvanically connected to said electrically conductive housing at an outer surface of said implantable medical device.

8. The implantable medical device of any of claims 1-7'
wherein said electrically conductive structure is wire or strip.

9. The implantable medical device of any of claims 1-8
wherein electrically conductive structure is made of a metallic material.

10. The implantable medical device of any of claims 1-9
wherein said electrically conductive structure (61) is meander shaped.

11. The implantable medical device of any of claims 1-10
wherein said electrically conductive structure is printed on a surface of said electrically insulating header portion.

12. The implantable medical device of any of claims 1-11
wherein said housing is hermetically sealed, and said antenna feeding is provided with a hermetic feed-through (13) in said housing.

## Patentansprüche

1. Implantierbare medizinische Vorrichtung, umfassend:
- ein elektrisch leitfähiges Gehäuse (1);
- eine elektrische Schaltungsanordnung für den Betrieb der implantierbaren medizinischen Vorrichtung und eine Funkfrequenz-Schaltungsanordnung zum Senden und/oder Empfangen von Funkfrequenzsignalen, wobei die elektrische Schaltungsanordnung und die Funkfrequenz-Schaltungsanordnung miteinander verbunden sind und innerhalb des elektrisch leitfähigen Gehäuses (1) angeordnet sind;
- einen elektrisch isolierenden Kopfabschnitt (3), welcher benachbart zu dem elektrisch leitfähigen Gehäuse angeordnet ist;
- eine elektrisch leitfähige Struktur (15, 45, 55, 61), die auf einer Fläche (3a) des und/oder innerhalb des elektrisch leitfähigen Kopfabschnitts (3) bereitgestellt ist;
- eine Antennenspeisung (9, 11), die betriebsfähig zwischen der Funkfrequenz-Schaltungsanordnung und der elektrisch leitfähigen Struktur geschaltet ist; und
- eine Durchgangsbohrung, die in einem ersten Endabschnitt (3b) des elektrisch isolierenden Kopfabschnitts (3) bereitgestellt ist und durch welche die Antennenspeisung (9, 11) und/oder ein kleinerer Abschnitt der elektrisch leitfähigen Struktur (45) geführt wird/werden, wobei
- die elektrisch leitfähige Struktur benachbart zu einem zweiten Endabschnitt (3c) des elektrisch isolierenden Kopfabschnitts galvanisch mit dem elektrisch leitfähigen Gehäuse verbunden ist, wobei sich der erste und zweite Endabschnitt gegenüberliegend zueinander befinden; und
- die elektrisch leitfähige Struktur und das elektrisch leitfähige Gehäuse gemeinsam angepasst sind, als Sende- und/oder Empfangsantenne für die Funkfrequenzsignale zu fungieren.

2. Implantierbare medizinische Vorrichtung nach Anspruch 1, wobei der elektrisch isolierende Kopfabschnitt (3) mindestens eine Öffnung (6) zur Aufnahme mindestens einer Anschlussleitung (5) umfasst und die elektrische Schaltungsanordnung mit der mindestens einen Anschlussleitung verbunden werden kann.

3. Implantierbare medizinische Vorrichtung nach Anspruch 2, wobei das Gehäuse hermetisch abgedichtet ist und in einer Wand des elektrisch leitfähigen Gehäuses eine hermetische Durchführung (7) bereitgestellt ist, um Verbindungen zu der mindestens einen Anschlussleitung (5) durch die Wand hindurchzuführen.

4. Implantierbare medizinische Vorrichtung nach einem der Ansprüche 1 bis 3, wobei zumindest ein größerer Abschnitt der elektrisch leitfähigen Struktur (15, 45, 61) auf der Fläche des elektrisch isolierenden Kopfabschnitts bereitgestellt ist.

5. Implantierbare medizinische Vorrichtung nach Anspruch 4, wobei es sich bei der Fläche des elektrisch isolierenden Kopfabschnitts, auf welcher der zumindest größere Abschnitt der elektrisch leitfähigen Struktur bereitgestellt ist, um eine äußere Fläche der implantierbaren medizinischen Vorrichtung handelt.

6. Implantierbare medizinische Vorrichtung nach einem der Ansprüche 1 bis 5, wobei die elektrisch leitfähige Struktur (45) im Wesentlichen in ihrer Gesamtheit auf der Fläche des elektrisch isolierenden Kopfabschnitts bereitgestellt ist.

7. Implantierbare medizinische Vorrichtung nach einem der Ansprüche 1 bis 6, wobei die elektrisch leitfähige Struktur an einer äußeren Fläche der implantierbaren medizinischen Vorrichtung galvanisch mit dem elektrisch leitfähigen Gehäuse verbunden ist.

8. Implantierbare medizinische Vorrichtung nach einem der Ansprüche 1 bis 7, wobei es sich bei der elektrisch leitfähigen Struktur um Draht oder Bandkabel handelt.

9. Implantierbare medizinische Vorrichtung nach einem der Ansprüche 1 bis 8, wobei die elektrisch leitfähige Struktur aus einem metallischen Material hergestellt ist.

10. Implantierbare medizinische Vorrichtung nach einem der Ansprüche 1 bis 9, wobei die elektrisch leitfähige Struktur (61) eine Mäanderform aufweist.

11. Implantierbare medizinische Vorrichtung nach einem der Ansprüche 1 bis 10, wobei die elektrisch leitfähige Struktur auf eine Fläche des elektrisch isolierenden Kopfabschnitts gedruckt ist.

12. Implantierbare medizinische Vorrichtung nach einem der Ansprüche 1 bis 11, wobei das Gehäuse hermetisch abgedichtet ist und die Antennenspeisung mit einer hermetischen Durchführung (13) in dem Gehäuse bereitgestellt ist.

## Revendications

1. Dispositif médical implantable comprenant :
- un boîtier électriquement conducteur (1) ;
- un circuit électrique servant à faire fonctionner ledit dispositif médical implantable et un circuit radiofréquence servant à émettre et/ou recevoir des signaux radiofréquence, ledit circuit électrique et ledit circuit radiofréquence étant interconnectés et disposés dans ledit boîtier électriquement conducteur (1) ;
- une partie d'embase électriquement isolante (3) disposée adjacente audit boîtier électriquement conducteur ;
- une structure électriquement conductrice (15, 45, 55, 61) prévue sur une surface (3a) de ladite et/ou à l'intérieur de ladite partie d'embase électriquement isolante (3) ;
- une alimentation d'antenne (9, 11) fonctionnellement interconnectée entre ledit circuit radiofréquence et ladite structure électriquement conductrice ; et
- un trou traversant, prévu dans une première partie d'extrémité (3b) de ladite partie d'embase électriquement isolante (3), par le biais duquel ladite alimentation d'antenne (9, 11) et/ou une partie mineure de ladite structure électriquement conductrice (45) est/sont guidée(s),
dans lequel
- ladite structure électriquement conductrice est reliée galvaniquement audit boîtier électriquement conducteur au voisinage d'une deuxième partie d'extrémité (3c) de ladite partie d'embase électriquement isolante, lesdites première et deuxième parties d'extrémité étant situées à l'opposé l'une de l'autre ; et
- ladite structure électriquement conductrice et ledit boîtier électriquement conducteur sont adaptés conjointement pour fonctionner comme une antenne d'émission et/ou de réception desdits signaux radiofréquence.

2. Dispositif médical implantable selon la revendication 1, dans lequel ladite partie d'embase électriquement isolante (3) comprend au moins une ouverture (6) destinée à recevoir au moins un fil (5) et ledit circuit électrique peut être relié audit au moins un fil.

3. Dispositif médical implantable selon la revendication 2, dans lequel ledit boîtier est scellé hermétiquement et une traversée étanche (7) est prévue dans une paroi dudit boîtier électriquement conducteur afin de guider des connexions vers ledit au moins un fil (5) à travers ladite paroi.

4. Dispositif médical implantable selon l'une quelconque des revendications 1 à 3, dans lequel au moins une partie majeure de ladite structure électriquement conductrice (15, 45, 61) est prévue sur la surface de ladite partie d'embase électriquement isolante.

5. Dispositif médical implantable selon la revendication 4, dans lequel la surface de ladite partie d'embase électriquement isolante sur laquelle ladite au moins une partie majeure de ladite structure électriquement conductrice est prévue est une surface extérieure dudit dispositif médical implantable.

6. Dispositif médical implantable selon l'une quelconque des revendications 1 à 5, dans lequel ladite structure électriquement conductrice (45) est prévue pratiquement en totalité sur la surface de ladite partie d'embase électriquement isolante.

7. Dispositif médical implantable selon l'une quelconque des revendications 1 à 6, dans lequel ladite structure électriquement conductrice est reliée galvaniquement audit boîtier électriquement conducteur au niveau d'une surface extérieure dudit dispositif médical implantable.

8. Dispositif médical implantable selon l'une quelconque des revendications 1 à 7, dans lequel ladite structure électriquement conductrice est un fil ou une bande.

9. Dispositif médical implantable selon l'une quelconque des revendications 1 à 8, dans lequel la structure électriquement conductrice est faite d'un matériau métallique.

10. Dispositif médical implantable selon l'une quelconque des revendications 1 à 9, dans lequel ladite structure électriquement conductrice (61) est de forme sinueuse.

11. Dispositif médical implantable selon l'une quelconque des revendications 1 à 10, dans lequel ladite structure électriquement conductrice est imprimée sur une surface de ladite partie d'embase électriquement isolante.

12. Dispositif médical implantable selon l'une quelconque des revendications 1 à 11, dans lequel ledit boîtier est scellé hermétiquement et ladite alimentation d'antenne est munie d'une traversée étanche (13) dans ledit boîtier.
